# EUROPEAN PATENT SPECIFICATION

(11) **EP 2 157 928 B1**
(45) Date of publication and mention of the grant of the patent: **07.01.2015**
(21) Application number: 07861963.2
(22) Date of filing: 13.11.2007
(51) Int. Cl.: A61H 7/00, A61B 18/14, A61B 18/20, A61N 1/00, A61B 17/00, A61B 18/00, A61N 7/00, A61N 1/40, A61N 5/06, A61H 9/00

(54) **APPARATUS, TIP AND METHOD FOR TREATING TISSUE**
GERÄT, SPITZE UND VERFAHREN ZUR BEHANDLUNG VON GEWEBE
APPAREIL, INSTRUMENT ET PROCÉDÉ PERMETTANT DE TRAITER UN TISSU

(30) Priority: 13.11.2006 US 858374 P; 13.11.2006 US 858391 P
(43) Date of publication of application: 03.03.2010
(73) Proprietor: Lumenis, Inc., Santa Clara, CA 95054 (US)
(72) Inventor: KHEN, Roee, Haifa (IL); ARIEL, Dan Alexandru, Kfar Saba (IL)
(74) Representative: Virdee-Crofts, Kulwinder Kaur
(86) International application number: PCT/US2007/023786
(87) International publication number: WO 2008/063478

(56) References cited:
- EP-A1- 1 627 662
- WO-A-03/005921
- WO-A-2005/060354
- WO-A-2007/117580
- GB-A- 1 417 989
- US-A1- 2004 077 977
- US-A1- 2005 251 118

## Description

### FIELD OF THE INVENTION

The present invention relates to methods, apparatus and tips for treating tissue, including cellulite-afflicted tissue.

### BACKGROUND OF THE INVENTION

Cellulite is a subcutaneous fat tissue that protrudes into the dermis, creating an undulating dermal-subcutaneous fat junction adipose tissue, which causes dimpling of skin. One procedure of removing excess adipose or fat tissue is liposuction. This is an invasive procedure in which the fat is destroyed mechanically and then extracted from under the skin using a suction device. As with any invasive surgical procedure, the procedure carries with it health risks to the patient. Moreover, liposuction is not effective for cellulite reduction.

EP1627662 and WO03/005921 disclose an apparatus for treating cellulitis by combined methods such as mechanical massage (using a vacuum treatment), heat extraction and laser systems for bio-stimulation.

### SUMMARY OF THE INVENTION

In one aspect, an apparatus for treating tissue is disclosed, as set out in claim 1. Embodiments of the apparatus may further include one or more of the following features. The one or energy transmitting elements may be configured to apply energy to at least a portion of the moved area of the tissue.

The apparatus may further include a vacuum pump coupled to the chamber that is configured to cause vacuum to be created in the chamber. The vacuum created in the chamber may cause the tissue to be raised towards the chamber.

The vacuum pump may be configured to cause a sequence of vacuum pulses to be created in the chamber. The vacuum pump may be configured to cause a sequence of vacuum pulses to be created in the chamber such that the periodic movement of the area of the tissue is performed.

The apparatus may further include a pump-control mechanism to control operation of the vacuum pump.

The apparatus may further include a filter mechanism to prevent entry into the vacuum pump of at least some substances disposed on the area of the tissue.

The apparatus may further include a vacuum solenoid valve configured to couple the chamber to one of the vacuum pump and air in an area outside the chamber such that when the vacuum solenoid couples the chamber to the vacuum pump and the vacuum pump is powered vacuum is created in the chamber, and when the vacuum solenoid couples the chamber to the area outside the chamber the air pressure level in the chamber converges to the air pressure level in the area outside the chamber

The one or more energy transmitting elements may be configured to apply to the portion of the raised area of the tissue one or more of, for example, Radio Frequency (RD) energy, ultrasound energy and/or energy and electrical pulses.

The one or more energy transmitting elements may include one or more light sources disposed in the chamber, the one or more light source may be configured to deliver light energy to the portion of the area of the tissue. The one or more light sources may include intense pulsed light (IPL) source.

The one or more energy transmitting elements may be arranged substantially perpendicular to the portion of the raised area of the tissue.

The one or more energy transmitting elements may be arranged substantially parallel to the portion of the raised area of the tissue.

The one or more energy transmitting elements may include one or more electrodes.

The one or more energy transmitting elements may be disposed proximate to the opening of the chamber.

The one or more energy transmitting elements may be configured to apply energy to the at least the portion of the raised area of the tissue when a computed vacuum level for the chamber exceeds a first threshold value and to terminate application of the energy to the at least the portion of the raised area of the tissue when the computed vacuum level for the chamber is at a second pre-determined threshold value.

The apparatus may further include a cooling sub-system to cool the apparatus and/or the tissue.

In another aspect, a cosmetic method is disclosed as set out in claim 13.

Embodiments of the method may include any of the features described above in relation to the apparatus, and also any of the following features.

The method may further include applying lotion to the tissue.

Applying energy to the portion of the raised area of the tissue may include determining air pressure level in the chamber, and applying the energy to the portion of the area of the tissue based on the determined air pressure level in the chamber.

Raising the area of the tissue towards the chamber may include actuating a valve having a first and second states into one of the first and second states, wherein when the valve is actuated to the first state vacuum is created in the chamber, and when the valve is actuated to the second state the air pressure level in the chamber converges to air pressure level in an area outside the chamber.

A treatment tip to treat tissue is also disclosed. The tip includes chamber having an opening, the chamber configured to, when placed proximate to the tissue, cause periodic movement of an area of the tissue raised towards the opening of the chamber, and one or more energy transmitting elements to apply energy to at least a portion of the area of the tissue.

Embodiments of the tip may include any of the features described above in relation to the apparatus and method.

The apparatus, treatment tip and method described herein enable non-invasive procedures for efficiently treating tissue, including cellulite-afflicted tissue, in a relatively easy, efficacious, and cost effective manner.

The principles and operation of embodiments of the system, apparatus, and use according to the present invention may be better understood with reference to the drawings, and the following description, it being understood that these drawings are given for illustrative purposes only and are not meant to be limiting. Other features, objects, and advantages of the invention will be apparent from the description and drawings, and from the claims.

### BRIEF DESCRIPTION OF THE DRAWINGS

FIG. 1 is a schematic of an exemplary embodiment of an apparatus to treat tissue.
FIG. 2A is a three-dimensional view of an exemplary embodiment of a treatment tip to treat tissue.
FIG. 2B is a cross-sectional view of an exemplary embodiment of the treatment tip of FIG. 2A. FIG. 2C is a cross-sectional view, from another angle, of the treatment tip of FIG. 2A
FIG. 2D is a three-dimensional view of a lower body part of the treatment tip of FIG. 2A. FIG. 3A is a graph depicting an exemplary embodiment of a sequence of vacuum pulses and corresponding RF pulses that are delivered to the target portion of a tissue.
FIG. 3B is a graph depicting another exemplary embodiment of a sequence of vacuum pulses and corresponding RF pulses that are delivered to the target portion of a tissue.
FIG. 4 is a flowchart of an exemplary embodiment of a procedure to treat of a tissue.
FIG. 5 is a schematic illustration of exemplary embodiments of roller configurations.

The dimensions of some of the elements may be exaggerated relative to other elements for clarity. Further, where considered appropriate, reference numerals may be repeated among the drawings to indicate corresponding or analogous elements throughout the views.

### DETAILED DESCRIPTION OF THE INVENTION

Disclosed herein are an apparatus, treatment tip and method for treatment of tissue, for example, cellulite-afflicted tissue. Other conditions and tissues may be treated using embodiments of the apparatus, tip and method disclosed herein. The apparatus, tip and method disclosed herein enable reducing cellulite in the treated tissue, using kneading, pressuring and/or stretching of the treated tissue, as well as by applying energy to the tissue so as to, for example, cause heat to be applied into the treated tissue, thus modifying, reducing or destroying undesirable cells in the treated tissue. Stretching of the treated tissue may, for example, decrease the depth of tissue and thus bring the afflicted cells closer to the surface of the tissue so as to facilitate the treatment of these cells.

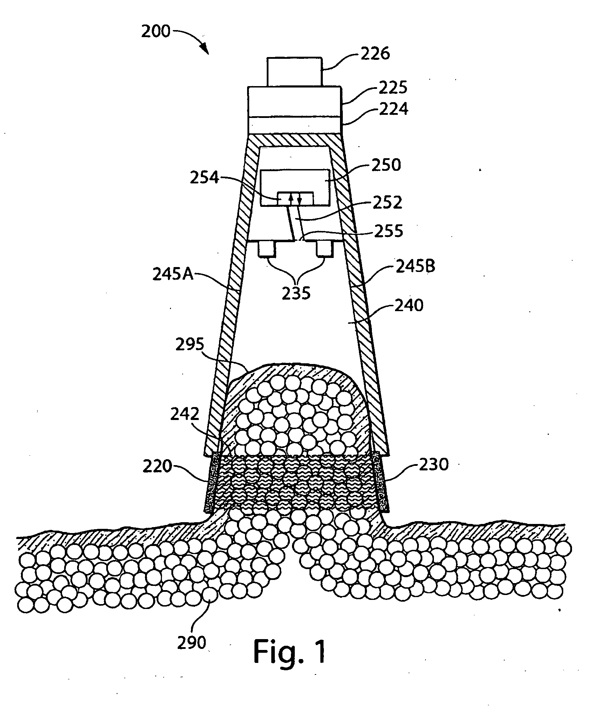

Referring to FIG. 1, an exemplary embodiment of an apparatus 200 to enable treatment of tissue 290 is shown. The apparatus 200 includes a vacuum chamber 240 defined by the interior walls 245A and 245B bounding the chamber. The chamber 240 includes an opening 242 (illustrated by dashed lines). The chamber 240 is configured to, when placed proximate to the tissue 290, to raise an area of the tissue towards the opening 242 of the chamber 240. As will be explained in greater detail below, in some embodiments the area of the tissue 290 is raised by creating a vacuum in the chamber 240 that causes the tissue to be drawn towards the chamber and through the opening 242. Furthermore, by creating a sequence of vacuum pulses in the chamber 240, stretching, kneading, massaging and other types of tissue movement can be performed.

As further shown in FIG.1, apparatus 200 includes one or more energy transmitting elements, for example, elements 220 and 230 (e.g., electrodes) that are coupled to and receive energy from an energy source 225 such as a battery, power supply, etc. The energy transmitting elements 220 and 230 are positioned proximate to the chamber 240, and may include an exposed section such as a planar section. In some embodiments, the planar sections of the energy transmitting elements face the inside of vacuum chamber 240 and are positioned proximate to the opening 242 of the chamber 240. The energy transmitting elements 220 and 230 may be positioned in other locations relative to the chamber 240 and may be arranged in different orientations relative to each other and/or to the chamber 240 or the tissue 290. For example, the elements 220 and 230 may be parallel to each other, as well as to the raised surface of the skin. Generally, the one or more energy transmitting elements 220 and 230 have a position and orientation such that when an area of the tissue 290 is raised (e.g., by creating a vacuum in the chamber 240) the tissue 290 comes in contact with the elements 220 and 230 to thus enable the elements 220 and 230 to apply energy to at least a portion of the raised area of the tissue. Energy transmitting elements 220 and 230 may be set parallel to each other or tilted at the proximate end toward each other at a desired angle, for example, an angle of about 60° to each other. In some embodiments, the elements 220 and 230 may be co-planar with each other and with the surface of the skin, for example, at an angle to chamber walls 245A and 245B.

Additionally and/or alternatively, the apparatus 200 may also include one or more light sources 235, disposed, in some embodiments, in the chamber 240 (e.g., opposite the opening 242) such that the one or more sources of light 235 illuminate a portion of the area of the tissue being treated to thus apply energy to that targeted portion. The light sources can includes, for example, intense pulse light (IPL) sources, coherent light sources (e.g., laser light sources) and non-coherent light sources. In some embodiments, light generated by the one or more light sources 235 is directed via suitable waveguides, e.g., optical fibers (not shown), that deliver the light to the targeted portions.

The apparatus 200 may include a vacuum pump 250 coupled to the chamber 240 via a pipe or conduit 252. The vacuum pump 250 is controlled to cause pulsed changes in air pressure, e.g., vacuum pulses, in the vacuum chamber 240. The vacuum pulses caused by the vacuum pump 250 may, for example, enable the performance of kneading, pressuring, stretching and/or other types of periodic movement of the tissue 290. The vacuum pump 250 causes air to be evacuated out of chamber 240, thus causing the tissue 290 to be raised, or drawn, towards chamber 240. In some embodiment, the energy transmitting elements 220 and 230 are configured to apply energy when the vacuum level exceeds a pre-determined vacuum level threshold (i.e., when the area of the tissue is sufficiently raised that it comes in contact with the energy transmitting elements), and to terminate application of energy after a pre-determined period of time has elapsed, or when the vacuum level in the chamber reaches another pre-determined vacuum level threshold. The control of the application of energy by the elements 220 and 230 can also be performed based on other measured characteristics. For example, the impedance between two points in the chamber 240 can be measured to determine the extent to which the area of the tissue 290 has been raised and is protruding into the volume defined by the chamber 240. Thus, if the impedance is above or below a certain impedance threshold level indicative that the tissue has been sufficiently raised into the volume of the chamber 240, the energy transmitting elements can commence applying energy to the targeted portion of the tissue. If the measured impedance is above or below some other pre-determined impedance threshold value, application of energy by the elements 220 and 230 can be terminated. In embodiments in which the light sources 235 are used to apply optical energy to the tissue being treated, a determination of the extent to which the area of the tissue being raised has been received in the volume defined by the chamber 240 can be performed by measuring optical properties associated with the chamber and the tissue 290. For example, the level of light reflection in the chamber 240 can indicate how much of the tissue 290 has been received in the chamber 240, thus controlling the operation of the light sources 235 to cause them to illuminate the portion of the light received in the chamber 240.

In some embodiments, the vacuum pump 250 is controlled by a control module 224 to actuate the vacuum pump 250 (e.g., to cause it to commence pumping operation to evacuate air from the chamber 240). For example, the control module 224 may include a processor-based device that can receive control input data, such as values indicative of pressure conditions within the chamber 240, and based on the received information generate signals to actuate the vacuum pump 250 (e.g., commence or terminate pumping operation of the pump). The control module 224 may also include pre-defined operation profiles, for example, a temporal profile specifying when the pump is to begin evacuating air from the chamber 240 and when it should cease pumping operation. Control of operation of the pump may be performed by the control module 224 generating actuating signal (e.g., electrical or mechanical signals) based on input data received by the module and/or pre-defined operation profiles, and sending the generated signals to the vacuum pump. Under these circumstances, the vacuum pump 250 would include an interface to receive the actuating signals.

The control module 224 may include a processor-based device that includes a computer and/or other types of processor-based devices suitable for multiple applications. Such devices can include volatile and non-volatile memory elements, and peripheral devices to enable input/output functionality. Such peripheral devices include, for example, a CD-ROM drive and/or floppy drive, or a network connection, for downloading related content. Such peripheral devices may also be used for downloading software containing computer instructions to enable general operation of the control module, and for downloading software implemented programs to perform operations to control the operation of the apparatus 200, including the operation of the vacuum pump 250.

A train or series of vacuum pulses may be activated and/or stopped, for example, manually, e.g., by a push-button (not shown). Alternatively and/or additionally, a vacuum solenoid valve 254 that can open or close the valve mechanically by passing current through the solenoid may be used to implement the formation of pulsating (e.g., cyclical) vacuum levels in the chamber 240. Solenoid valve 254 may be actuated to switch between at least two states by using, for example, by a control module such as the control module 224, or by using a dedicated control module (not shown). In a first state, solenoid valve 254 may couple the vacuum pump 250 to vacuum chamber 240. In this first state, activation of the vacuum pump 250 may result in negative pressure or vacuum in the chamber 240, thus causing suction action to be performed by the chamber 240 to raise the tissue 290 towards the chamber 240. When actuated to a second state the solenoid valve 254 may connect vacuum chamber 240 to the outside environment, thus allowing the air pressure inside the chamber 240 to converge towards the air pressure level outside the apparatus 200 (e.g., the ambient air pressure).

As noted, in some embodiments, when the tissue 290 is raised towards the chamber, and sufficient contact is achieved between the energy transmitting elements 220 and 230 and the portion of the area of the tissue that was substantially received in the volume defined by the chamber 240, energy pulses (e.g., (radio frequency (RF) energy) are applied to the tissue via energy transmitting elements 220 and 230. The energy thus applied can result in heat being provided to at least that portion, as well as to other portions that neighbor that portion that has in come in contact with the energy transmitting elements, which in turn enables treating and/or alleviating various conditions of the tissue (e.g., cellulite).

Generally, the energy transmitting elements 220 and 230 apply energy to the portion of the raised area of the tissue 290 when sufficient contact has been established between the portion of the raised area of the tissue and the elements 220 and 230, or otherwise when the targeted portion to which the energy is to be applied is sufficiently close the energy transmitting elements 220 and 230. The elements 220 and 230 generally do not apply energy when the target portion of the tissue 290 is not sufficient close or is not in contact with the electrode, thus conserving energy, improving the efficacy of the treatment performed by the apparatus 200 and reducing any medical risks associated with applying energy when the targeted portion is not sufficiently close, or in contact, with the energy transmitting elements 220 and 230. To determine whether there is sufficient contact, or sufficient proximity, between treated tissue and the elements 220 and 230, a computed pressure level, indicative of the vacuum level in the chamber 240, is determined. Thus, a pressure sensor or gauge (not shown) may be disposed in the chamber 240 to measure the pressure within the chamber. Based on the measured pressured value, the energy transmitting elements apply energy to the targeted portion of the raised area of the tissue. For example, if the computed pressure (or vacuum) level is determined to exceed some pre-determined first threshold value, thus indicating that the area of the tissue 290 may be sufficiently raised, and accordingly, the targeted portion of the raised area may be sufficiently close or in contact with the energy transmitting elements 220 and 230, energy is applied to the portion of the raised area via the energy transmitting elements 220 and 230. Conversely, if the computed pressure/vacuum level in the chamber 240 is determined to be below or above some pre-determined threshold value (a threshold value that may be the same or different from the first threshold value) application of energy by the energy transmitting elements 220 and 230 is either terminated (in the event that the elements had been applying energy up to that point) or not commenced.

In some embodiments, energy transmitting elements 220 and 230 may apply other forms of energy, including, for example, ultrasound, intense pulsed light (IPL), or electrical pulses. Under those circumstances, suitable energy transmitting elements configured to generate and/or apply those other types of energy are used. Appropriate energy source or energy generation module corresponding to those different types energy are coupled to the energy transmitting elements of the type being used. For example, in circumstances in which intense pulse light is to be applied to the treated tissue 290 via the elements 220 and 230, the apparatus 200 would include an IPL source and the energy transmitting elements could include lenses and optical filters to direct received light pulses to the targeted portion of the raised area of the tissue.

In some embodiments, the apparatus may have at least two sets of energy sources, with each energy source connected to a set of one or more energy transmitting elements suitable for transmitting that energy. For example, an RF energy source may be used in concert with an intense pulsed light (IPL) source, with each source delivering generated energy to appropriate energy transmitting elements (e.g., electrodes for the RF energy source and lenses and filters to direct received light to the target portion of the tissue). As another example, in some embodiments, the apparatus 200 device may include an RF-based energy source, and corresponding energy transmitting elements, that operate in concert with an ultrasound energy source operating in concert with a dedicated set of one or more suitable energy transmitting elements (e.g., transducers). In embodiments in which the apparatus 200 includes more than one energy source, the device may have a first mode in which energy sources may operate substantially simultaneously on the tissue, and a second mode in which energy sources may operate alternatingly.

Energy transmitting elements 220 and 230 may be conducting electrodes. In some embodiments, elements 220 and 230 may comprise a material that may prevent skin burning. For example, elements 220 and 230 may comprise copper, plastic coated with a conductive coating, brass, stainless steel, aluminum, gold coating or combinations thereof. A material such as a gel, lotion, cream, or other suitable substance may be applied to the surface of treated section 295 of the tissue 290, for example, to facilitate easy stretching and/or expansion of the treated section 295 into vacuum chamber 240 and/or increase conductivity of the treated section 295. The vacuum pump 250 may include a filter 255 to protect vacuum pump 250 from undesirable substances, such as loose lotion particles, skin debris, etc., that may be drawn into the chamber 240.

In some embodiments, apparatus 200 may include a user interface that includes, for example, a touch screen allowing selection and alteration of parameters of the vacuum pulses e.g., minimum and maximum vacuum levels, period time of vacuum pulses, discharge duration as a percentage from the period (e.g., off-duty cycle), sufficient levels for energy application, rest duration between pulses, and other parameters germane to the operation of the apparatus 200 and performance of the sequence of vacuum pulses. The user interface may further allow for alteration of parameters of the applied energy such as, for example, power level, frequency, and other parameters of the applied energy.

The apparatus 200 may further include rollers (not shown in FIG. 1). A description of the use of roller with a tissue treating apparatus is provided for example, in pending U.S. patent application serial No. 11/937,917, entitled "Apparatus and Method for Treating Tissue" and filed November 9, 2007, published as US 2008 221504. Generally, the rollers may, for example, facilitate drawing and/or expansion of the treated tissue into the vacuum chamber 240, thus, for example, preventing excessive friction or tearing of the tissue when the apparatus is moved along the tissue or when the tissue is drawn into the vacuum chamber 240. The rollers may thus enable easy sliding of apparatus 200 on the surface of the tissue 290 from one section of the tissue to another without removing the apparatus 200 from the tissue. Therefore, for example, the treatment of a large area may be continuous and uninterrupted. The rollers may be installed, for example, in a way that seals vacuum chamber 240, for example, by inhibiting air-flow between the vacuum chamber 240 and the air outside of the apparatus 200. The rollers can be disposed on springs and configured to close into (i.e., pinch) the area of the tissue 290 drawn into the vacuum chamber 240. In some embodiments, the apparatus 200 includes an electro-mechanical assembly configured to move the rollers inward and outward across the opening of the vacuum chamber 240. The motion of the rollers can be coordinated with the level of pressure within the vacuum chamber 240 to create a squeezing or massaging motion as the tissue moves into and out of the chamber 240.

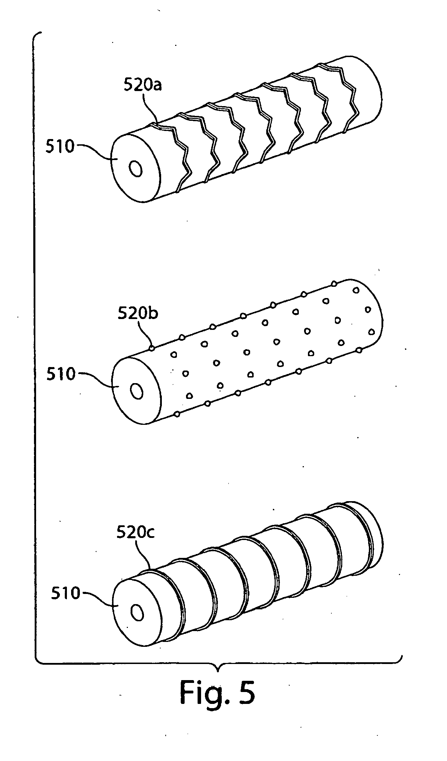

In some embodiments, the surface of the rollers may be partially non-conducting. For example, the rollers may be divided into sections, for example, two outer sections and an inner section. The outer sections, e.g., near the ends of the rollers, may have coating from rubber or another non-conducting material. Referring to FIG. 5, in some embodiments, the rollers 510 include alternating bands of conducting 520a, b, c, materials configured to apply energy in patterns as the apparatus 200 is moved along the tissue 290. For example, the rollers 510 include conductive 520a, b, c material protruding up from on a non-conductive material in a fixed pattern, such as a herringbone, dots, or dashes. The relative size and location of the conductive and non-conductive materials can be varied based on the treatment regime. In some embodiments, the roller 510 is conductive and includes non-conductive protrusions 520b, c. In general, the roller materials can be high in friction when in contact with the skin, such that the device can roll with the tissue and not slip or slide thereupon. In some embodiments, the conducting portion of the rollers may not be entirely smooth, for example, they may have protrusions or the surface of the electrode may be roughened. As an example, and not a limitation, protrusions include small teeth similar to a gear assembly spaced around the roller, scoring etched around the roller, bumps or small hollow needles (e.g., micro-fabricated micro needles) disposed on the roller.

In some embodiments, the apparatus 200 may further include a cooling sub-system 226, to provide cooling for the components of the apparatus 200 and/or of the tissue 290. For example, the cooling sub-system 226 may cool energy transmitting elements 220 and 230, for example to protect the higher resistance stratum cornea, which is a layer of cells on the outermost layer of the epidermis. The cooling sub-system 226 may additionally and/or alternatively cool the skin on the surface of the tissue 290, and/or the upper levels of the tissue etc. A variety of suitable cooling sub-systems may be used, for example, thermal electric cooling mechanisms, water-cooling mechanisms, gas-cooling mechanisms, or other suitable cooling mechanisms. The cooling sub-system 240 may be associated with the tissue, energy transmitting elements, etc. Further detail regarding the cooling sub-system 226 and of cooling of the apparatus 200 and/or the tissue 290 is provided, for example, in U.S. Patent No. 7,250,047, entitled "System and Method for Treating Tissue".

In some embodiments, the apparatus 200 may further include a tissue-massaging unit (not shown) configured to massage the tissue during, for example, operation of the pump 250 and/or the energy transmitting elements 220 and 230.

In some embodiments, the tissue raising and energy application operations may be performed using an attachable/detachable treatment tip that may be secured, through a suitable interfacing mechanism, to an external system. Use of an attachable/detachable tip enables replacing different tips, by attaching them to the external system, when performing different tissue treatment procedures and/or when performing tissue treatment procedures on different patients.

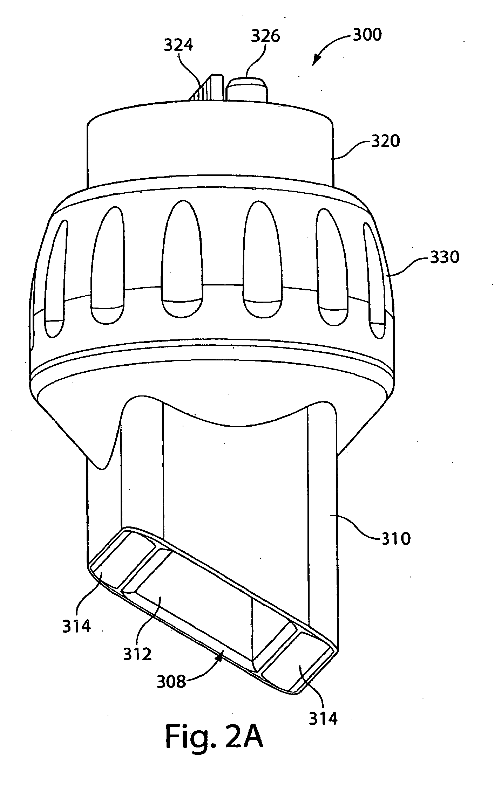

FIGS. 2A-2D provide a detailed illustration of the structural arrangement of an embodiment of a treatment tip 300. The treatment tip 300 may be an attachable tip that can be attached to an external system that includes, for example, a pump, an energy source and/or a control module.

Referring to FIG. 2A, a view of an exemplary embodiment of the treatment tip 300 is shown. The treatment tip 300 may include a lower body part 310 and an upper body part 320. The treatment tip 300 may further include a nut 330 that may be secured to the upper body part 320. The nut 330 may reinforce a connection between the lower body part 310 and the upper body part 320. The lower body part 310 includes a chamber 312, which may be similar to the chamber 240 shown and described in relation to FIG. 1. The chamber 312 includes an opening 308 which may be placed over the treated tissue (not shown in FIG. 2A) so that the bottom edges of the walls defining the chamber 312 and the opening 308 are either contacting the tissue or are close to it. As explained in relation to the chamber 240 of FIG. 1, the chamber 312 of FIG. 2A is also configured to cause an area of the tissue to be drawn, or raised, towards it so that part of the tissue enters through the opening and is disposed in the volume of the chamber 312. To raise the area of the tissue toward the chamber 312, a vacuum may be created in the chamber 312. Furthermore, the treatment of the tissue may include periodic stretching, kneading, massaging, and other types of periodic movement variations performed on the area of the tissue, and thus a vacuum pulses sequence may be created in the chamber 312 to cause periodic physical movement the area of the tissue being treated.

As further shown, the lower body part 310 of the tip 300 also includes one or more energy transmitting elements 314 that may be similar to the one or more energy transmitting elements 220 and 230 shown in FIG. 1. The one or more energy transmitting elements 314 are configured to apply energy to a portion of the raised area of the tissue, for example, to provide heat to the tissue.

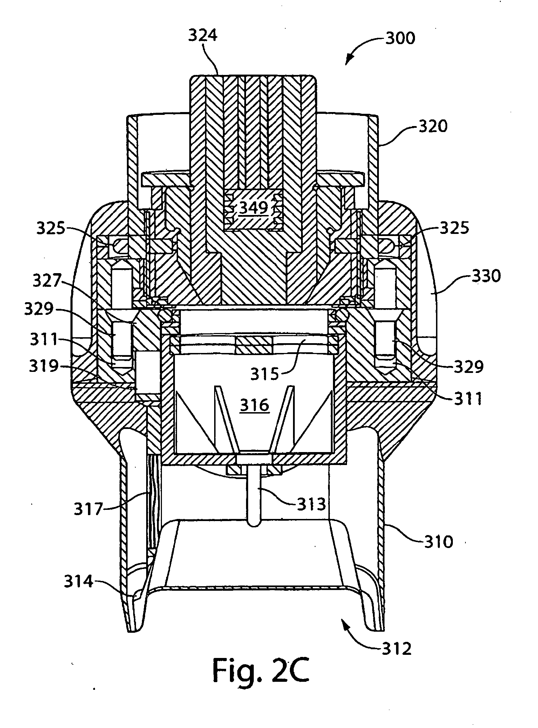

The upper body part 320 generally includes an attachment mechanism to attach the tip 300 to the external system. In some embodiments, the upper body part 320 may include an electronic board 324. The electronic board 324 may be connected to a control module (not shown) to control the operation of treatment tip 300. Such a control module may be disposed in the external system (e.g., disposed in a head section of the external system having an interface to enable attachment of the tip 300 thereto). The electronic board 324 may also be connected to an energy source (not shown) such as the energy source(s) of the apparatus 200. The energy source and control module connected to the upper body 320 may be includes in the external system to which the treatment tip 300 is attached. The electronic board 324 may conduct energy to the energy-transmitting elements 314 via electrodes 325 (shown, for example, in FIG. 2C). The upper body part 320 may include a lumen 326 that is coupled to a vacuum pump (not shown). Like the control module and energy source, the vacuum pump may also be disposed externally to the tip 300 and can be connected through a suitable interfacing mechanism. The lumen 326 may be in mechanical communication with a filter to prevent infiltration of foreign bodies, such as skin debris into the vacuum pump.

The treatment with the tip 300 may include applying of a lotion or gel on the treated tissue to, for example, facilitate displacement of the treatment tip 300 on the treated tissue, to facilitate, for example, stretching of the tissue and/or increase conductivity of the treated tissue. To prevent infiltration of lotion or gel or other foreign substances into to vacuum pump and/or to other parts of the system, filters may be installed in treatment tip 300.

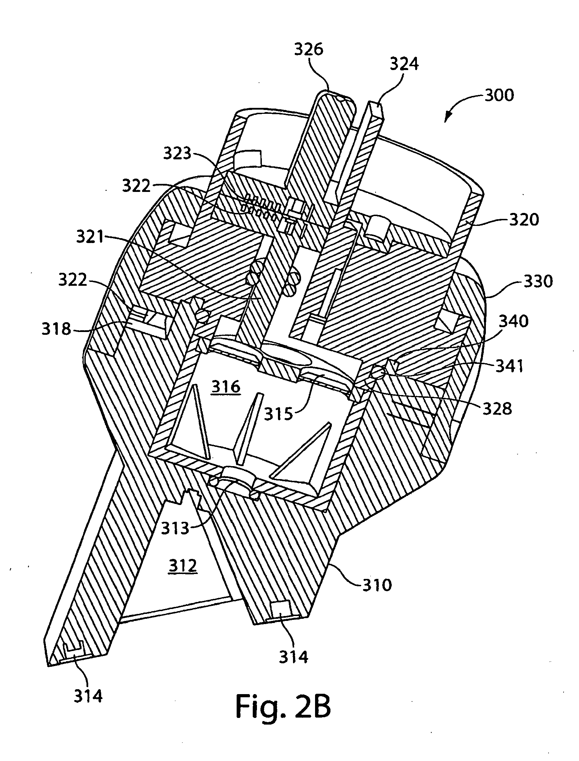

Referring to FIG. 2B, a cross-sectional view of the treatment tip 300 is shown. The lower body part 310 may include a channel 313 through which air may be pumped out of the chamber 312. The lower body part 310 may also include a collection chamber 316, in which lotion, gel, or other foreign substances that entered the chamber 312 when air was being evacuated may be deposited. The lower body part 310 may include filtering sections 315 which may prevent infiltration of lotion, gel and other foreign substances into other parts of the treatment tip 300. The filtering sections may be made of, for example, filter paper or any other suitable filtering material. The upper body part 320 may include a lower filter part 322 and an upper filter part 323. The lower filter part 322 and the upper filter part 323 may filter the pumped air after passing through the collection chamber 316 and the filtering sections 315. The lower filter part 322 may be in communication with a pipe 321 through which the air is pumped into the lower filter part 322 and the upper filter part 323. The upper filter part 323 may be in communication with the lumen 326, which may in turn be connected to the vacuum pump.

In some embodiments, the lower body part 310 may include grooves 318 to, for example, enable connection of the nut 330 to the lower body part 310. The nut 330 may include pins 332 configured to slide into the grooves 318 thus connecting the nut 330 to the lower body part 310 by, for example, a bayonet connection. The upper body part 320 may be connected to the lower body part 310 by a snap ring 328. The snap ring 328 may be snapped into an O-ring 341 inside a ring 340 of the lower body part 310. The upper body part 320 may be further connected to the lower body part 310 by pins 329 (shown in FIG. 2C).

In some embodiments, the tip 300 may also include a solenoid valve similar in structure and functionality to the solenoid valve 254 of the apparatus 200 shown in FIG. 1.

Referring to FIG. 2C, a cross-sectional view, as seen from another angle, of the treatment tip 300. The upper body part 320 may include pins 329 configured to connect the upper body part 320 to the lower body part 310. The lower body part 310 may include grooves 311 into which the pins 329 are inserted, thus connecting the upper body part 320 to the lower body part 310. The upper body part 320 may also include the conductors 325 connected to electronic board 324. The energy transmitting elements 314 may be connected to wires 317 which in turn may extend through the lower body part 310 until they reach the contact points 319. The conductors 325 may extend through the upper body part 320 until contact points 327. When the upper body part 320 and the lower body part 310 are connected, an electrical path is established between the contact points 319 and the contact points 327, thus connecting the energy transmitting elements 314 to the electronic board 324. The electronic board 324 may include an electronic chip 349 to collect, for example, data regarding the operation of the treatment tip 300 (e.g., vacuum levels in the chamber 312) and to transfer data to the control module (not shown) controlling the operation of the treatment tip 300.

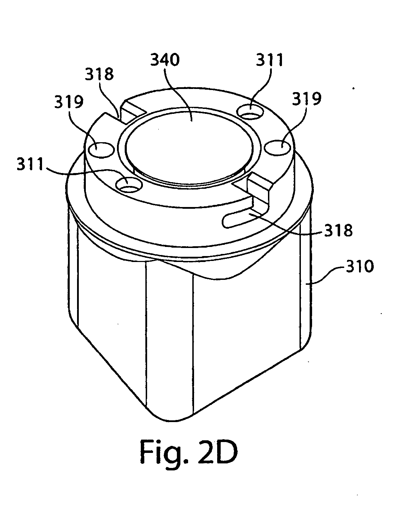

Referring to FIG. 2D, a three-dimensional view of the lower body part 310 of the treatment tip 300 is shown. As described herein, the lower body part 310 may include the grooves 318. In some embodiments, the grooves 318 may be curved thus enabling a connection of the nut 330 to the lower body part 310, e.g., by inserting the pins 332 into the grooves 318 and turning the nut 330 so that pins 332 may slide into the curve of grooves 318.

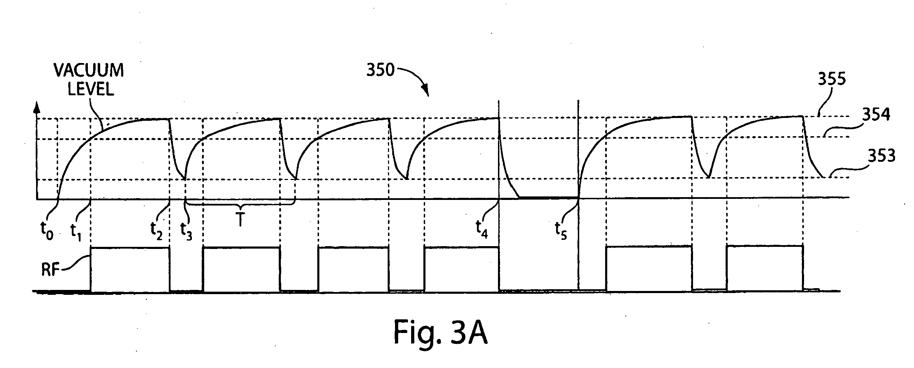

Referring to FIG. 3A, a graph of an exemplary embodiment of a sequence of vacuum pulses 350 and corresponding RF pulses that can be delivered by an apparatus, such as apparatus 200, and/or by a system that includes a treatment tip such as the tip 300, is shown. The sequence of vacuum pulses (i.e., cyclical variations of the air pressure in the chamber 240) may be implemented, for example, using the solenoid valve 254 as described herein, and by using a control module to generate actuating signals that control the operation of, for example, the vacuum pump 250 and the solenoid valve 254.

Particularly, as shown in FIG. 3A, at time *t*₀, a vacuum pulse may be commence, and solenoid switch may be set so as to allow the vacuum pump to evacuate air out of the vacuum chamber (e.g., the vacuum chamber 240).

At time *t*₁ the vacuum level inside the vacuum chamber may exceed a sufficient level 354 (alternatively, the air pressure in the chamber may be determined to have fallen below a corresponding threshold value). The pre-defined vacuum threshold level 354 may be considered to be the vacuum level at which sufficient contact between the energy transmitting elements 220 and 230 and the portion of the raised area of the treated tissue is deemed to exist. When the vacuum level in the chamber 240 reaches or exceeds the threshold vacuum level 354, an energy source, e.g., an RF source, may be activated to provide energy to the energy transmitting elements 220 and 230 which can then direct the energy, in an appropriate form, to the treated tissue. The duration of the pulse (e.g., RF pulse) may be pre-determined, for example, by a knob or other setting indicating a user preference, or by a selection by a software-implemented user interface. The duration of the RF pulse may alternatively and/or additionally be specified in a pre-defined operation profile stored in, for example, the control module 224.

The duration of the RF pulse (e.g., 200 milliseconds) may correspond to the point at which the vacuum level in the chamber is to be decreased (i.e., air pressure is increased), thus releasing the raised area of tissue. Particularly, at the end of the application of the RF pulse (i.e., at time *t*₂), the solenoid valve 254 may be actuated to be set, for example, to its second state in which no additional air is evacuated from the chamber 240 and/or air.is introduced into the chamber 240 from, for example, an area outside the chamber 240.

The air evacuation stage of every pulse may stop at some pre-determined time e.g., every 200 milliseconds, at which point, the vacuum-decreasing stage (i.e., the stage at which the tissue is being released) of the pulse may be commenced. In other words, once it is determined that a pre-defined time period (e.g., 200 milliseconds) has elapsed, application of energy by the energy transmitting electrode to the portion of the raised area of the tissue will be terminated. Alternatively, the air evacuation stage may end when it is determined that the vacuum level in the chamber 240 has reached some pre-determined maximum vacuum level threshold value. For example, in FIG. 3A the air evacuation stage may end when it is determined that the computed vacuum level in the chamber 240 has reached the maximum vacuum level threshold 355, e.g., approximately 28 inHg. In some embodiments, the RE energy source may cease providing energy to the tissue when maximum level 355 is reached.

When the solenoid valve is actuated to its second state, in which the air flow out of the chamber 240 is prevented and/or air is introduced into the chamber 240, the vacuum level may reach a minimum level 353 at *t*₃. The minimum level 353 may correspond to, for example, approximately 30% of the maximum level 355. In some embodiments the threshold level 354 may be set, for example, to approximately 70% of the maximum level 355.

Upon reaching the minimum vacuum level 353, the current vacuum pulse (cycle) is completed, and the next vacuum pulse can begin. Accordingly, the cyclical process in which air is evacuated from the chamber 240 and an energy pulse is applied to a portion of a raised area of the tissue when the vacuum level reaches the pre-defined threshold 54 is repeated, as depicted in the vacuum pulses following the time *t*₃ in FIG. 3A. This process may thereafter repeat so that a pulsating vacuum cycle with a period T results. In some embodiments, the period *T* of each pulse may be, for example, about 1 second. The release duration *t*₃-*t*₂ may be predetermined according to the desired minimum level 353. At the end of each cycle in the sequence of pulses, i.e., when a minimum vacuum level such as level 353, is reached, the vacuum pump may be activated again (and/or the solenoid valve may be actuated to its other state) thus enabling air flow between the vacuum pump and the chamber 240 so that air can be evacuated from the chamber 240. Generally, in each cycle of the vacuum pulse sequence, when the vacuum level in the chamber 240 reaches the pre-defined threshold level 354, the one or more energy transmitting elements may commence applying energy to a portion of the raised area of the tissue until, for example, the vacuum level in the chamber 240 reaches the maximum vacuum threshold value 355.

The repetition of vacuum pulses may continue until, at time *t*₄, the vacuum pulses may be stopped, for example, based on a pre-programmed setting of number of pulses, or based on input provided by a user of the apparatus 200 (e.g., the user turning an ON/OFF switch to stop operation of the apparatus 200). At some later time, *t*₅, after a pause, which may be pre-programmed or may be controlled in real-time by the user (e.g., by toggling an ON/OFF switch of the apparatus 200), the vacuum pulse sequence may resume. The pause in the application of vacuum pulses and energy pulses may enable the transfer of the chamber 240 to another area of the tissue so that that other area can now be treated using the apparatus 200.

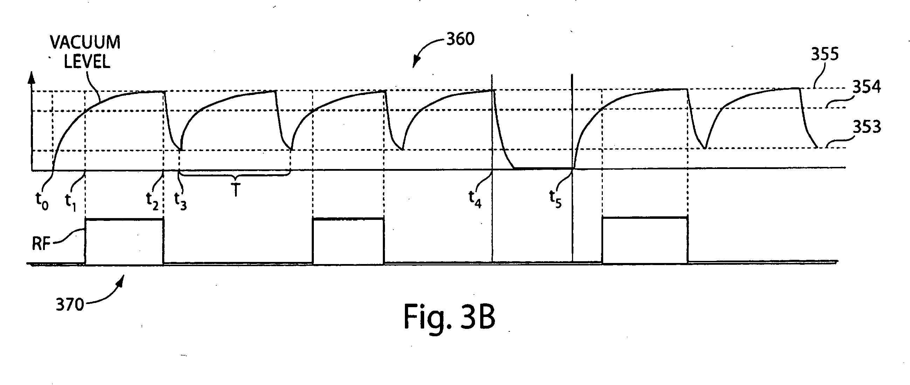

Referring to FIG. 3B, an exemplary embodiment of a sequence 360 of vacuum pulses and their corresponding RF pulses 370 resulting from operation of the apparatus 200 is shown. As depicted in FIG. 3B, the sequence 360 may, for example, include the application of an energy pulse (e.g., an RF energy pulse) during every other vacuum pulse, rather than with every vacuum pulse (as performed in the embodiment of the vacuum pulse sequence 350 of FIG. 3A). Other combinations of RF pulses and vacuum pulses may be provided. For example, an RF pulse may be provided during every third vacuum pulse or every fourth vacuum pulse, etc.

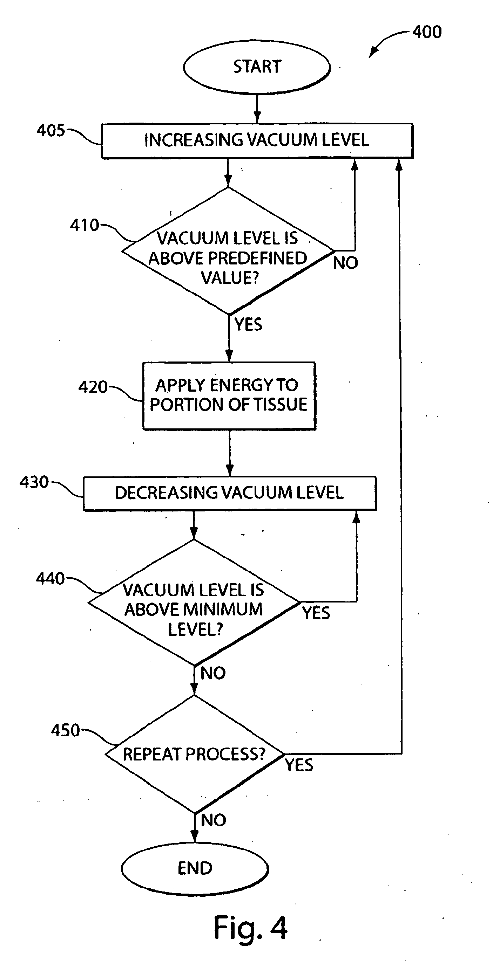

Referring to FIG. 4, a flowchart depicting an exemplary procedure 400 to treat tissue is shown. After placing the opening 242 of the chamber 240 (or the opening 308 of the chamber 312 of the treatment tip 300) proximate to the area of the tissue 290 to be treated, the vacuum pump 250 may be powered using the power source 225 to power the vacuum pump 250. A user operating the apparatus 200 may control in real-time the operation of the apparatus 200, including the operation of the vacuum pump vacuum pulse sequence and energy pulses that are applied during the vacuum pulse cycles. Alternatively, once the apparatus is turned on and directed to a desired area of the tissue 290, the application of the vacuum pulses sequence and the energy pulse sequence may be controlled using a control module, such as the control module 224, that may generate signals to actuate the vacuum pump 250 and/or the solenoid valve 254.

Once the pump 250 is activated and a cycle of the vacuum pulse sequence is commenced, the pump 250 evacuates air from the chamber 240, thus increasing 405 the vacuum level in the chamber 240. The evacuation of air from the chamber 240 causes an area of the tissue 290 to be raised towards the chamber 240 through suction operation. The vacuum level in the chamber is measured to determine 410 if the vacuum level has exceeded a pre-defined vacuum threshold level. If it the threshold has not yet been exceeded, the vacuum level in the chamber 410 is increased (e.g., by further evacuation of air).

On the other hand, when the vacuum level threshold has been exceeded, and thus sufficient contact, or sufficient proximity, between the one or more energy transmitting elements and the targeted portion of the of the raised area of the tissue is deemed to exist, the energy transmitting elements 220 and 230 begin to apply energy 420 (e.g., RF energy, IPL energy, etc.) to the portion of the raised area of the tissue 290. The application of energy to the targeted portion of the raised area of the tissue 290 may continue, in some embodiments, for a specified period of time (e.g., 200 milliseconds) or until a pre-defined vacuum level in the chamber 240 is reached. In some embodiments, while the energy transmitting elements apply energy to the targeted portion, the increase of the vacuum level in the chamber 240 may continue (operation not shown in the flowchart of FIG. 4).

Once the energy applied to the target area has stopped, or alternatively, once the maximum vacuum level in the chamber 240 has been reached, the vacuum level in the chamber 240 is decreased 430. Decreasing the vacuum level in the chamber 240 can be performed by, for example, actuating the vacuum pump 250 so that it ceases evacuation of air from the chamber 240, actuating the vacuum pump 250 so that it reverses its operation to pump air into the chamber 240 and/or actuating the solenoid valve 254 to enable air, e.g., air from an area outside the chamber 240, enter the chamber 240 to thus cause the air pressure level in the chamber 240 to converge to the air pressure level in the area outside the chamber 240.

As the vacuum level is being decreased in the chamber 240, the vacuum level is measured to determine 440 if the vacuum level in the chamber 240 has reached a minimum vacuum level threshold. If it has not (i.e., the vacuum level in the chamber 240 is above that minimum threshold), the vacuum level in the chamber continues to be decreased.

Once the vacuum level in the chamber 240 is no longer above the minimum threshold (i.e., it has reached the minimum vacuum level threshold), it is determined 450 whether the procedure 400 is to be repeated to thus begin another cycle of the vacuum pulse sequence. As described herein, in some embodiments, the vacuum pulse sequence will performed according to a pre-specified number of cycles that are to be repeated. In some embodiments, a user can terminate the vacuum pulse sequence by indicating so through a user-interface input device (e.g., a knob or a switch). Termination of the vacuum pulse sequence enables the re-positioning of the chamber 240 at another area of the tissue 290 that is to be treated and/or replacement of an attachable tip similar to the tip 300.

A number of embodiments of the invention have been described. Nevertheless, it will be understood that various modifications may be made without departing from the scope of the invention as claimed. Accordingly, other embodiments are within the scope of the following claims.

## Claims

1. An apparatus (200) for treating tissue (290), the apparatus (200) comprising:
a control module (224)
a vacuum pump (250) adapted to be coupled to a chamber (240, 312), to evacuate the chamber (240, 312);
said chamber (240, 312) having an opening (242,308), whereby, when placed proximate to tissue (290), an area of the tissue (290) can be raised through the opening (242,308) into the chamber (240); and
one or more energy transmitting elements (220, 230, 314) disposed in the chamber (240, 312);
the control module (224) is responsive to at least one of;
(a) a computed vacuum level for the chamber (240) exceeding a first threshold;
(b) an impedance measured between two points in the chamber (240); and/or
(c) measured optical properties associated with the chamber (240) and the raised tissue (290);
which indicate that the tissue (290) is determined to be sufficiently drawn into the chamber, to actuate an energy transmitting element (220, 230, 314) and begin to apply energy to at least a portion of the tissue (290),
**characterized in that** a valve (254) being responsive to said control module (224), to control the chamber vacuum pressure to apply and relieve the vacuum in a sequence of vacuum pulses and
said control module (224) is responsive to a subsequent level of the at least one of any of the computed vacuum, the impedance or measured optical properties to discontinue the application of energy by the energy transmitting element (220, 230, 314).

2. The apparatus according to claim 1 wherein the valve (254) is a solenoid valve.

3. The apparatus of claim 1 or claim 2, wherein the one or more energy transmitting elements (220, 230, 314) are configured to apply one or more of:
Radio Frequency (RF) energy, ultrasound energy, electro-optical energy and electrical pulses.

4. The apparatus of any one of the preceding claims, wherein the one or more energy transmitting elements (220, 230, 314) comprise one or more light sources (235) disposed in the chamber, the one or more light sources configured to deliver light energy to the at least the portion of the area of the tissue (290).

5. The apparatus of any of claims 1 to 4, comprising: a replaceable tip (300) configured to be attached to and detached from an external system, the replaceable tip (300) comprising:
the chamber (312) having an opening (308), and
the one or more energy transmitting elements (314) to apply energy to at least the portion of the area of the tissue.

6. The apparatus of claim 5, comprising:
an attachment mechanism to attach the tip (300) to the external system, the external system comprising a head section configured to be attached to tip (300), the head section includes at least one of: the vacuum pump (250), the control module (224) and an energy source (225) to provide energy to the one or more energy transmitting elements (314).

7. The apparatus of claim 5 or 6 wherein the tip (300) comprises:
a lower body part (310) and an upper body part (320), said lower body part (310) including the chamber (312) and opening (308) and connectable to the upper body part via a bayonet fitting.

8. The apparatus of any one of claims 5 to 7, wherein the energy transmitting elements (314) include one or more light sources configured to deliver light energy to the area of the tissue (290).

9. The apparatus of any one of the preceding claims comprising:
one or more rollers (510), arranged to seal the vacuum chamber (240, 312) and facilitate drawing and/or expansion of the treated tissue (290) into the vacuum chamber (240, 312).

10. The apparatus of claim 9 comprising the rollers (510) configured to pinch the area of the tissue (290) drawn into the vacuum chamber (240, 312).

11. The apparatus of claim 9 or 10 wherein the apparatus (200) includes an electro-mechanical assembly configured to move the rollers (510) inward and outward across the opening (242, 308) of the vacuum chamber (240, 312) to create a squeezing and/or massaging motion.

12. The apparatus of any of claims 9-11 wherein the rollers (510) include alternating bands of conducting materials (520a, b, c,) configured to apply energy in patterns as the apparatus (200) is moved along the tissue (290).

13. A cosmetic method of treating cellulite for the purpose of cosmetic improvement comprising the steps of:
applying an opening (242,308) of a chamber (240,312) to an area of dermis tissue with cellulite intrusion (290),
periodically cyclically evacuating the chamber (240,312) to draw the area of the cellulite dermal tissue (290) into the chamber (240,312) and charging the chamber (240,312) to expel the area of tissue in order to cause periodic movement of the area of the tissue (290); and
using a control module (224) responding to at least one of:
(a) a computed vacuum level for the chamber (240,312) exceeding a first threshold;
(b) an impedance measured between two points in the chamber (240,312); and/or
(c) measured optical properties associated with the chamber (240,312) and the raised tissue (290);
to determine that the area of the tissue (290) is sufficiently raised at a threshold (t1) and to actuate energy transmitting units to begin to apply energy to at least a portion of the area of the tissue (290) drawn into the chamber (240,312) and to discontinue the energy application when the tissue is lowered below a threshold (t2).

14. The cosmetic method of claim 13, wherein applying energy to the area of the dermal tissue comprises: applying light energy through one or more light sources (235) disposed in the chamber (240,312).

15. The cosmetic method of one of claims 13 or 14, wherein raising the area of the tissue (290) towards the chamber (240,312) comprises:
actuating a valve (254) into one of a first state and a second state, wherein when the valve (254) is actuated to the first state the chamber (240,312) is evacuated, and when the valve (254) is actuated to the second state the air pressure level in the chamber (240,312) converges to air pressure level in an area outside the chamber.

## Patentansprüche

1. Vorrichtung (200) zum Behandeln eines Gewebes (290), wobei die Vorrichtung (200) Folgendes umfasst:
ein Steuermodul (224);
eine Vakuumpumpe (250), die dazu eingerichtet ist, mit einer Kammer (240, 312) verbunden zu werden, um die Kammer (240, 312) zu evakuieren;
wobei die Kammer (240, 312) eine Öffnung (242, 308) hat, wobei, wenn sie in der Nähe eines Gewebes (290) platziert wird, ein Bereich des Gewebes (290) durch die Öffnung (242, 308) in die Kammer (240) angehoben werden kann; und
eine oder mehrere Energieübertragungselemente (220, 230, 314), die in der Kammer (240, 312) angeordnet sind;
wobei ein Ventil (254) auf das Steuermodul (224) reagiert, um den Kammervakuumdruck zu steuern, um das Vakuum in einer Abfolge von Vakuumimpulsen anzulegen und zu entspannen, und
**dadurch gekennzeichnet, dass** das Steuermodul (224) auf mindestens eines der folgenden reagiert:
(a) ein berechnetes Vakuumniveau für die Kammer (240), das einen ersten Grenzwert überschreitet;
(b) eine Impedanz, die zwischen zwei Punkten in der Kammer (240) gemessen wird; und/oder
(c) gemessene optische Eigenschaften, die mit der Kammer (240) und dem angehobenen Gewebe (290) in Zusammenhang stehen;
die anzeigen, dass von dem Gewebe (290) bestimmt wird, dass es ausreichend in die Kammer gesogen wird, um ein Energieübertragungselement (220, 230, 314) zu betätigen und damit zu beginnen, Energie auf mindestens einen Teil des Gewebes (290) anzuwenden,
wobei das Steuermodul (224) auf ein anschließendes Niveau des mindestens einen von einem beliebigen des berechneten Vakuums, der Impedanz oder der gemessenen optischen Eigenschaften reagiert, um das Anwenden von Energie durch das Energieübertragungselement (220, 230, 314) zu unterbrechen.

2. Vorrichtung nach Anspruch 1, wobei das Ventil (254) ein Magnetventil ist.

3. Vorrichtung nach Anspruch 1 oder 2, wobei das eine oder die mehreren Energieübertragungselemente (220, 230, 314) dazu konfiguriert sind, eine oder mehrere der folgenden anzuwenden: Radiofrequenzenergie (RF-Energie), Ultraschallenergie, elektrooptische Energie und elektrische Impulse.

4. Vorrichtung nach einem der vorhergehenden Ansprüche, wobei das eine oder die mehreren Energieübertragungselemente (220, 230, 314) eine oder mehrere Lichtquellen (235) umfassen, die in der Kammer angeordnet sind, wobei die eine oder die mehreren Lichtquellen dazu konfiguriert sind, Lichtenergie an mindestens den Teil des Bereichs des Gewebes (290) abzugeben.

5. Vorrichtung nach einem der Ansprüche 1 bis 4, die Folgendes umfasst: eine austauschbare Spitze (300), die dazu konfiguriert ist, an einem externen System angebracht und von diesem getrennt zu werden, wobei die austauschbare Spitze (300) Folgendes umfasst:
die Kammer (312) mit einer Öffnung (308) und
das eine oder die mehreren Energieübertragungselemente (314), um Energie auf mindestens den Teil des Bereichs des Gewebes anzuwenden.

6. Vorrichtung nach Anspruch 5, die Folgendes umfasst:
einen Anbringungsmechanismus, um die Spitze (300) an dem externen System anzubringen, wobei das externe System einen Kopfabschnitt umfasst, der dazu konfiguriert ist an der Spitze (300) angebracht zu werden, wobei der Kopfabschnitt mindestens eines der folgenden aufweist: die Vakuumpumpe (250), das Steuermodul (224) und eine Energiequelle (225), um dem einen oder den mehreren Energieübertragungselementen (314) Energie bereitzustellen.

7. Vorrichtung nach Anspruch 5 oder 6, wobei die Spitze (300) Folgendes umfasst:
ein unteres Körperteil (310) und ein oberes Körperteil (320), wobei das untere Körperteil (310) die Kammer (312) und die Öffnung (308) aufweist und mittels eines Bajonettverschlusses mit dem oberen Körperteil verbunden werden kann.

8. Vorrichtung nach einem der Ansprüche 5 bis 7, wobei die Energieübertragungselemente (314) eine oder mehrere Lichtquellen aufweisen, die dazu konfiguriert sind, Lichtenergie an den Bereich des Gewebes (290) abzugeben.

9. Vorrichtung nach einem der vorhergehenden Ansprüche, die Folgendes umfasst:
eine oder mehrere Walzen (510), die dazu eingerichtet sind, die Vakuumkammer (240, 312) abzudichten und das Ansaugen und/oder Ausdehnen des behandelten Gewebes (290) in der Vakuumkammer (240, 312) zu erleichtern.

10. Vorrichtung nach Anspruch 9, die die Walzen (510) umfasst, die dazu konfiguriert sind, den Bereich des Gewebes (290), der in die Vakuumkammer (240, 312) gesogen wird, zusammenzudrücken.

11. Vorrichtung nach Anspruch 9 oder 10, wobei die Vorrichtung (200) eine elektromechanische Baugruppe aufweist, die dazu konfiguriert ist, die Walzen (510) nach innen und nach außen durch die Öffnung (242, 308) der Vakuumkammer (240, 312) zu bewegen, um eine Drück- und/oder Massageaktion zu erzeugen.

12. Vorrichtung nach einem der Ansprüche 9 -11, wobei die Walzen (510) alternierende Streifen aus leitenden Materialien (520a, b, c) aufweisen, die dazu konfiguriert sind, Energie in Mustern anzuwenden, während die Vorrichtung (200) entlang dem Gewebe (290) bewegt wird.

13. Kosmetisches Verfahren zum Behandeln von Cellulitis zum Zwecke einer kosmetischen Verbesserung, das die folgenden Schritte umfasst:
Anbringen einer Öffnung (242, 308) einer Kammer (240, 312) an einem Bereich von Lederhautgewebe mit eingedrungener Cellulitis (290),
periodisches zyklisches Evakuieren der Kammer (240, 312), um den Bereich des Cellulitis-Lederhautgewebes (290) in die Kammer (240, 312) zu saugen, und Befüllen der Kammer (240, 312), um den Gewebebereich auszustoßen, um eine periodische Bewegung des Bereichs des Gewebes (290) zu bewirken; und
Verwenden eines Steuermoduls (224), das auf mindestens eines der folgenden reagiert:
(a) ein berechnetes Vakuumniveau für die Kammer (240, 312), das einen ersten Grenzwert überschreitet;
(b) eine Impedanz, die zwischen zwei Punkten in der Kammer (240, 312) gemessen wird; und/oder
(c) gemessene optische Eigenschaften, die mit der Kammer (240, 312) und dem angehobenen Gewebe (290) in Zusammenhang stehen;
um zu bestimmen, dass der Bereich des Gewebes (290) mit einem Grenzwert (t1) ausreichend angehoben wird, und um Energieübertragungseinheiten dahingehend zu betätigen, damit zu beginnen, Energie auf mindestens einen Teil des Bereichs des Gewebes (290), der in die Kammer (240, 312) gesogen wird, anzuwenden und die Energieanwendung zu unterbrechen, wenn das Gewebe unter einen Grenzwert (t2) gesenkt wird.

14. Kosmetisches Verfahren nach Anspruch 13, wobei das Anwenden von Energie auf den Bereich des Lederhautgewebes Folgendes umfasst: Anwenden von Lichtenergie durch eine oder mehrere Lichtquellen (235), die in der Kammer (240, 312) angeordnet sind.

15. Kosmetisches Verfahren nach einem der Ansprüche 13 oder 14, wobei das Anheben des Bereichs des Gewebes (290) in Richtung der Kammer (240, 312) Folgendes umfasst:
Betätigen eines Ventils (254) in einen eines ersten Zustands und eines zweiten Zustands, wobei, wenn das Ventil (254) in den ersten Zustand betätigt wird, die Kammer (240, 312) evakuiert wird, und wenn das Ventil (254) in den zweiten Zustand betätigt wird, das Luftdruckniveau in der Kammer (240, 312) sich einem Luftdruckniveau in einem Bereich außerhalb der Kammer annähert.

## Revendications

1. Appareil (200) destiné à traiter un tissu (290), l'appareil (200) comprenant :
un module de commande (224) ;
une pompe à vide (250) conçue pour être couplée à une chambre (240, 312), afin d'évacuer la chambre (240, 312) ;
ladite chambre (240, 312) possédant une ouverture (242, 308) laquelle fait que, lorsqu'elle est placée à proximité d'un tissu (290), une zone de tissu (290) peut être soumise à un relèvement à travers l'ouverture (242, 308) pour l'amener dans la chambre (240) ; et
un ou plusieurs éléments de transmission d'énergie (220, 230, 314) lesquels sont disposés dans la chambre (240, 312) ;
une soupape (254) laquelle réagit audit module de commande (224), afin de piloter la pression sous vide dans la chambre en vue d'appliquer et de relâcher le vide suivant une séquence d'impulsions de vide, et
**caractérisé en ce que** le module de commande (224) réagit à l'un au moins des postes suivants, à savoir :
(a) un niveau de vide calculé pour la chambre (240) lequel dépasse un premier seuil ;
(b) une impédance mesurée entre deux points dans la chambre (240) ; et/ou
(c) des propriétés optiques mesurées lesquelles sont associées à la chambre (240) et au tissu relevé (290) ;
lesquels indiquent qu'il a été déterminé que le tissu (290) est suffisamment aspiré dans la chambre pour actionner un élément de transmission d'énergie (220, 230, 314) et pour commencer à appliquer de l'énergie à au moins une portion du tissu (290),
ledit module de commande (224) réagissant à un niveau ultérieur dudit au moins un poste parmi l'un quelconque des postes suivants, à savoir le vide calculé, l'impédance ou les propriétés optiques mesurées afin de faire cesser l'application d'énergie par l'élément de transmission d'énergie (220, 230, 314).

2. Appareil selon la revendication 1, la soupape (254) étant une soupape à solénoïde.

3. Appareil selon la revendication 1 ou la revendication 2, lesdits un ou plusieurs éléments de transmission d'énergie (220, 230, 314) étant configurés de façon à appliquer un ou plusieurs des postes suivants, à savoir : énergie de radiofréquence (RF), énergie ultrasonique, énergie électro-optique et impulsions électriques.

4. Appareil selon l'une quelconque des revendications précédentes, lesdits un ou plusieurs éléments de transmission d'énergie (220, 230, 314) comprenant une ou plusieurs sources lumineuses (235) lesquelles sont disposées dans la chambre, lesdites une ou plusieurs sources lumineuses étant configurées de façon à délivrer de l'énergie lumineuse à au moins ladite portion de la zone de tissu (290).

5. Appareil selon l'une quelconque des revendications 1 à 4, comprenant : un embout remplaçable (300) configuré pour être attaché à un système externe, et pour en être détaché, l'embout remplaçable (300) comprenant :
la chambre (312) avec une ouverture (308), et
lesdits un ou plusieurs éléments de transmission d'énergie (314) afin d'appliquer de l'énergie à au moins la portion de la zone de tissu.

6. Appareil selon la revendication 5, comprenant :
un mécanisme de fixation pour attacher l'embout (300) au système externe, le système externe comportant une section tête configurée pour être attachée à l'embout (300), la section tête incluant au moins l'un des postes suivants, à savoir : la pompe à vide (250), le module de commande (224) et une source d'énergie (225) afin de fournir de l'énergie auxdits un ou plusieurs éléments de transmission d'énergie (314).

7. Appareil selon la revendication 5 ou 6, l'embout (300) comprenant :
une partie corps inférieur (310) et une partie corps supérieur (320), ladite partie corps inférieur (310) incluant la chambre (312) et l'ouverture (308) et apte à être raccordée à la partie corps supérieur à l'aide d'un raccord à baïonnette.

8. Appareil selon l'une quelconque des revendications 5 à 7, les éléments de transmission d'énergie (314) incluant une ou plusieurs sources lumineuses lesquelles sont configurées pour délivrer de l'énergie lumineuse à la zone de tissu (290).

9. Appareil selon l'une quelconque des revendications précédentes, comprenant :
un ou plusieurs rouleaux (510) agencés de façon à rendre étanche la chambre à vide (240, 312) et à faciliter l'aspiration et/ou l'expansion du tissu traité (290) dans la chambre à vide (240, 312).

10. Appareil selon la revendication 9, comprenant les rouleaux (510) configurés de façon à pincer la zone de tissu (290) aspirée dans la chambre à vide (240, 312).

11. Appareil selon la revendication 9 ou 10, l'appareil (200) incluant un ensemble électro-mécanique configuré de façon à déplacer les rouleaux (510) vers l'intérieur et vers l'extérieur à travers l'ouverture (242, 308) de la chambre à vide (240, 312) afin de créer un mouvement de compression et/ou de massage.

12. Appareil selon l'une quelconque des revendications 9 à 11, les rouleaux (510) incluant des bandes alternantes de matériaux conducteurs (520a, b, c) configurés de façon à appliquer de l'énergie suivant certains schémas, au fur et à mesure que l'appareil (200) est déplacé le long du tissu (290).

13. Procédé cosmétique de traitement de la cellulite à des fins d'une amélioration cosmétique, comprenant les étapes consistant à :
appliquer une ouverture (242, 308) d'une chambre (240, 312) sur une zone de tissu du derme avec une intrusion de cellulite (290),
périodiquement évacuer la chambre (240, 312) suivant un mode cyclique afin d'aspirer la zone de tissu dermique à cellulite (290) jusque dans la chambre (240, 312), et charger la chambre (240, 312) pour chasser la zone de tissu en vue de provoquer un mouvement périodique de la zone de tissu (290) ; et
utiliser un module de commande (224) lequel réagit à l'un au moins des postes suivants, à savoir :
(a) un niveau de vide calculé pour la chambre (240, 312) lequel dépasse un premier seuil ;
(b) une impédance mesurée entre deux points dans la chambre (240, 312) ; et/ou
(c) des propriétés optiques mesurées lesquelles sont associées à la chambre (240, 312) et au tissu relevé (290) ;
afin de déterminer que la zone de tissu (290) est suffisamment relevée jusqu'à un certain seuil (t1) et d'actionner des unités de transmission d'énergie pour commencer à appliquer de l'énergie à au moins une portion de la zone de tissu (290) aspirée jusque dans la chambre (240, 312) et cesser l'application d'énergie quand le tissu est abaissé en dessous d'un certain seuil (t2).

14. Procédé cosmétique selon la revendication 13, l'application d'énergie à la zone de tissu dermique comprenant l'opération consistant à : appliquer de l'énergie lumineuse grâce à une ou plusieurs sources lumineuses (235) disposées dans la chambre (240,312).

15. Procédé cosmétique selon l'une des revendications 13 ou 14, le relèvement de la zone de tissu (290) vers la chambre (240, 312) comprenant l'opération consistant à :
actionner une soupape (254) pour l'amener dans l'un des états suivants, à savoir un premier état et un second état, cas dans lequel lorsque la soupape (254) est actionnée vers le premier état, la chambre (240, 312) est évacuée, et lorsque la soupape (254) est actionnée vers le second état le niveau de pression d'air dans la chambre (240, 312) converge vers un niveau de pression d'air présent dans une zone à l'extérieur de la chambre.
